# EUROPEAN PATENT APPLICATION

(11) **EP 4 744 646 A1**
(43) Date of publication of application: **20.05.2026**
(21) Application number: 24840110.1
(22) Date of filing: 11.07.2024
(51) Int. Cl.: A61K 8/34, A61K 8/02, A61K 8/87, A61K 8/81, A61Q 19/08

(54) **COMPOSITION FOR ADJUSTING SKIN ADHESION FORCE AND KIT FOR SKIN ADHESION SHEET COMPRISING SAME**

(30) Priority: 13.07.2023 KR 20230091272; 10.07.2024 KR 20240091154
(71) Applicant: Amorepacific Corporation, Seoul 04386 (KR)
(72) Inventor: PI, Bongsoo, Yongin-si, Gyeonggi-do 17074 (KR); KIM, Jihoon, Yongin-si, Gyeonggi-do 17074 (KR); SEO, Jeongeun, Yongin-si, Gyeonggi-do 17074 (KR); SEO, Jeong Hye, Yongin-si, Gyeonggi-do 17074 (KR); HAN, Kyungsup, Yongin-si, Gyeonggi-do 17074 (KR); KIM, Soyeon, Yongin-si, Gyeonggi-do 17074 (KR)
(74) Representative: Schön, Christoph
(86) International application number: PCT/KR2024/009945
(87) International publication number: WO 2025/014300

(57) **Abstract**

Disclosed in the present specification are: a composition capable of adjusting the adhesive force and/or detachment force of a skin adhesion sheet comprising polyol; and a kit for a skin adhesion sheet comprising same, wherein the composition can increase an adhesion maintenance time of a patch while minimizing skin irritation when the skin adhesion sheet is detached.

## Description

### [Technical Field]

### [Cross-Reference to Related Application]

The present application claims priority to Korean Patent Application No. 10-2023-0091272, filed on July 13, 2023 and Korean Patent Application No. 10-2024-0091154, filed on July 10, 2024, the entire contents of which are incorporated herein by reference.

The present specification discloses a composition for adjusting a skin adhesion force, which may reduce irritation upon detachment of a skin adhesion sheet from skin while increasing adhesion maintenance time, and a kit for a skin adhesion sheet comprising the same.

### [Background Art]

A patch used in the pharmaceutical and cosmetic industries is generally classified into a wet type, in which a liquid preparation is impregnated into a non-woven fabric; a dry type, in which a freeze-dried collagen sheet contains an active ingredient; and a gel type, which includes both a hydrophilic gel and a lipophilic gel.

For cosmetic use, the hydrophilic gel and wet type are most widely used, while for medical use, the dry type and lipophilic gel, having excellent efficacy and consumer recognition, are frequently used.

Here, in the case of a dry or wet-type skin adhesion sheet attached to skin, a composition capable of exhibiting excellent adhesive force while minimizing skin irritation upon detachment from skin is required.

### [Disclosure]

### [Technical Problem]

In one aspect of the present disclosure, it is intended to provide a composition for adjusting a skin adhesion force, which may increase adhesion maintenance time while reducing irritation upon detachment of a skin adhesion sheet, and a kit for a skin adhesion sheet including the composition.

### [Technical Solution]

In one aspect, the present specification provides a composition for adjusting a skin adhesion force, in which the composition is for adjusting an adhesive force and/or a detachment force of a skin adhesion sheet attached to skin, and the composition includes, based on the total weight of the composition, 5 to 30 wt% of polyol and 60 wt% or less of water.

In another aspect, the present specification provides a kit for a skin adhesion sheet, which includes the composition described above and a skin adhesion sheet, and is for use by applying the composition onto skin and then attaching the skin adhesion sheet onto the composition.

### [Advantageous Effects]

In one aspect, by using the composition for adjusting a skin adhesion force disclosed in the present specification together with a skin adhesion sheet, the adhesive force may be enhanced while skin irritation upon detachment may be minimized.

### [Description of Drawings]

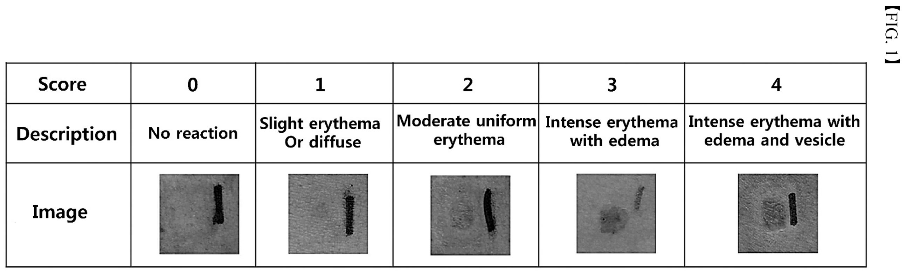

FIG. 1 is a result of measuring the degree of skin irritation in one embodiment of the present disclosure.

### [Mode for Disclosure]

Hereinafter, the present disclosure will be described in more detail with reference to the following embodiments. However, the following embodiments are provided for illustrative purposes only to assist in understanding the present disclosure and are not intended to limit the scope and range of the present disclosure.

In exemplary embodiments of the present disclosure, as a composition for adjusting a skin adhesion force, the composition is for adjusting an adhesive force and/or a detachment force of a skin adhesion sheet attached to skin, and the composition provides a composition including, based on the total weight of the composition, 5 to 30 wt% of polyol and 60 wt% or less of water.

In other exemplary embodiments of the present disclosure, a method for adjusting an adhesive force and/or a detachment force of a skin adhesion sheet is provided, the method including applying the composition for adjusting a skin adhesion force described above onto the skin adhesion sheet attached to skin.

In other exemplary embodiments of the present disclosure, a use of polyol and water for preparing a composition for adjusting a skin adhesion force is provided, in which the composition for adjusting a skin adhesion force includes, based on the total weight of the composition for adjusting a skin adhesion force, 5 to 30 wt% of polyol and 60 wt% or less of water, and the composition for adjusting a skin adhesion force is for adjusting an adhesive force and/or a detachment force of a skin adhesion sheet attached to skin.

In other exemplary embodiments of the present disclosure, a composition for adjusting a skin adhesion force for use in adjusting an adhesive force and/or a detachment force of a skin adhesion sheet attached to skin is provided, and the composition for adjusting a skin adhesion force includes, based on the total weight of the composition for adjusting a skin adhesion force, 5 to 30 wt% of polyol and 60 wt% or less of water.

In other exemplary embodiments of the present disclosure, a use of polyol and water for adjusting a skin adhesion force is provided, in which the adjusting of skin adhesion force is for adjusting an adhesive force and/or a detachment force of a skin adhesion sheet attached to skin, and the polyol and water are provided in a form of a composition, and the composition includes, based on the total weight of the composition, 5 to 30 wt% of polyol and 60 wt% or less of water.

In other exemplary embodiments of the present disclosure, a non-therapeutic use of a composition for adjusting a skin adhesion force for adjusting an adhesive force and/or a detachment force of a skin adhesion sheet attached to skin is provided, and the composition for adjusting a skin adhesion force includes, based on the total weight of the composition for adjusting a skin adhesion force, 5 to 30 wt% of polyol and 60 wt% or less of water.

The composition for adjusting a skin adhesion force according to one embodiment of the present disclosure is a solubilized formulation, and while conventional oil-in-water or water-in-oil formulations contain oil and thus have reduced adhesion force, the composition according to one embodiment of the present disclosure including polyol and water as main components may be easily attached to skin for a long period of time.

For example, adjusting an adhesive force and/or a detachment force of the skin adhesion sheet attached to skin may be a method for enhancing the adhesive force of the skin adhesion sheet while suppressing skin irritation upon detachment.

For example, a content of the polyol may be, based on the total weight of the composition for adjusting a skin adhesion force, 5 wt% or more, 10 wt% or more, 15 wt% or more, 20 wt% or more, or 25 wt% or more, and may be 30 wt% or less, 25 wt% or less, 20 wt% or less, or 15 wt% or less. When the polyol is less than 5 wt%, there may be a concern that skin irritation occurs upon detachment after skin adhesion, and when it exceeds 30 wt%, an adhesive force between the skin and the sheet may be weak.

For example, a content of the water may be, based on the total weight of the composition for adjusting a skin adhesion force, 10 wt% or more, 15 wt% or more, 20 wt% or more, 25 wt% or more, 30 wt% or more, 35 wt% or more, 40 wt% or more, 45 wt% or more, 50 wt% or more, or 55 wt% or more, and may be 60 wt% or less, 55 wt% or less, 50 wt% or less, 45 wt% or less, 40 wt% or less, 35 wt% or less, 30 wt% or less, 25 wt% or less, 20 wt% or less, or 15 wt% or less.

In one embodiment, a weight ratio of the polyol and the water may be 3:1 to 1:9. For example, the weight ratio of the polyol and the water may be 3:1 or more, 2:1 or more, 1:1 or more, 1:2 or more, 1:3 or more, 1:4 or more, 1:5 or more, 1:6 or more, 1:7 or more, or 1:8 or more, and may be 1:9 or less, 1:8 or less, 1:7 or less, 1:6 or less, 1:5 or less, 1:4 or less, 1:3 or less, 1:2 or less, 1:1 or less, or 2:1 or less.

In one embodiment, the polyol may be one or more selected from the group consisting of glycerin, butylene glycol, propylene glycol, and propanediol.

In one embodiment, the composition may further include a general thickener, a surfactant, a preservative, and a fragrance, and may further include natural moisturizing ingredients such as trehalose, betaine, hyaluronic acid, and beta-glucan.

In one embodiment, the composition may further include water (distilled water), EDTA-2Na, betaine, carbomer, 1,2-hexanediol, ethylhexylglycerin, tromethamine, beta-glucan, prehalose, green tea extract, sodium hyaluronate, shea butter, cetyl alcohol, xanthan gum, glyceryl stearate citrate, squalane, and the like.

In one embodiment, the composition may be a cosmetic composition.

The cosmetic composition according to the present disclosure may further include functional additives and ingredients included in general cosmetic compositions. The functional additive may include an ingredient selected from the group consisting of water-soluble vitamins, oil-soluble vitamins, polymeric peptides, polymeric polysaccharides, squalane, sphingolipids, and seaweed extracts.

The cosmetic composition according to the present disclosure may also be blended, together with the functional additive, with ingredients included in general cosmetic compositions as necessary. Additional blending ingredients may include oily components, moisturizers, emollients, emulsifiers, organic and inorganic pigments, organic powders, ultraviolet absorbers, preservatives, bactericides, antioxidants, plant extracts, pH adjusters, alcohols, colorants, fragrances, blood circulation promoters, cooling agents, antiperspirants, purified water and the like.

In one embodiment, the composition may be a skin external application composition.

The skin external application composition is a generic term including any formulation applied externally to skin, and various formulations of cosmetics may be included therein.

In one embodiment, the composition may be a pharmaceutical composition.

In one embodiment, the composition may be for prevention, improvement, or treatment of skin-related diseases.

In one embodiment, the composition may be for prevention, improvement, or treatment of inflammatory diseases.

As used herein, the term "prevention" refers to any act of suppressing a disease or delaying the onset of a disease by administration of the pharmaceutical composition according to the present disclosure.

As used herein, the term "improvement" refers to any act of reducing at least the degree of a parameter related to the condition being treated, for example, the severity of symptoms.

As used herein, the term "treatment" refers to any act of alleviating or beneficially changing symptoms caused by a disease by administration of the pharmaceutical composition according to the present disclosure.

In one embodiment, the composition for adjusting a skin adhesion force is for adjusting an adhesive force of the skin adhesion sheet, and when the skin adhesion sheet is attached onto the composition for adjusting a skin adhesion force, the adhesive force of the skin adhesion sheet may be 100 N/m or more. For example, the adhesive force may be 100 N/m or more, and may be 300 N/m to 400 N/m.

In one embodiment, the composition for adjusting a skin adhesion force may be for maintaining an adhesion maintenance time of the skin adhesion sheet for 1 hour or longer. For example, the adhesion maintenance time may be 1 hour or more, 2 hours or more, 3 hours or more, 4 hours or more, 5 hours or more, 6 hours or more, 7 hours or more, 8 hours or more, 9 hours or more, or 10 hours or more, and may be 24 hours or less, 20 hours or less, 16 hours or less, 12 hours or less, 10 hours or less, 9 hours or less, 8 hours or less, 7 hours or less, 6 hours or less, 5 hours or less, 4 hours or less, 3 hours or less, or 2 hours or less.

In one embodiment, the composition for adjusting a skin adhesion force may be for suppressing skin irritation upon detachment of the skin adhesion sheet, and the skin irritation upon detachment of the skin adhesion sheet may be 1 or less.

In one embodiment, the composition for adjusting a skin adhesion force may be used in an amount of 0.05 to 2 ml per 1 cm³ of the skin adhesion sheet.

For example, a use amount of the composition for adjusting a skin adhesion force may be 0.05 ml or more, 0.1 ml or more, 0.15 ml or more, 0.5 ml or more, 0.75 ml or more, 1 ml or more, 1.25 ml or more, 1.5 ml or more, or 1.75 ml or more, and may be 2 ml or less, 1.75 ml or less, 1.5 ml or less, 1.25 ml or less, 1 ml or less, 0.75 ml or less, 0.5 ml or less, 0.25 ml or less, 0.15 ml or less, or 0.1 ml or less per 1 cm³ of the skin adhesion sheet.

In one embodiment, the composition for adjusting a skin adhesion force may be applied to skin before attaching the skin adhesion sheet to the skin.

In one embodiment, the composition for adjusting a skin adhesion force may be applied to the skin within 5 seconds before attaching the skin adhesion sheet to the skin.

For example, the composition for adjusting a skin adhesion force may be applied to the skin within 5 seconds, within 4 seconds, within 3 seconds, within 2 seconds, or within 1 second before attaching the skin adhesion sheet to the skin, or may be applied to the skin immediately when attaching the skin adhesion sheet to the skin.

In one embodiment, the skin adhesion sheet may include a dry adhesive layer.

In one embodiment, the dry adhesive layer may include one or more selected from the group consisting of one or more hydrophilic polymers, one or more amine coupling groups, one or more crosslinkers, a coating agent, and a viscosity adjuster.

For example, the one or more hydrophilic polymers may be selected from the group consisting of polyacrylic acid, polyacrylamide, polyvinyl alcohol, polyhydroxy ethyl methacrylate, polyethylene glycol, polyvinylpyrrolidone, polystyrene sulfonate, casein, albumin, gelatin, collagen, chitosan, hyaluronic acid, alginate, oxidized alginate, pectin, and combinations thereof.

For example, the one or more hydrophilic polymers may include polyvinyl alcohol and hyaluronic acid.

For example, the amine coupling group may be selected from the group consisting of N-hydroxysuccinimide ester, N-hydroxy sulfosuccinimide ester, aldehyde, imidoester, epoxide, isocyanate, catechol, and combinations thereof.

For example, the crosslinker may be selected from the group consisting of gelatin methacrylate, hyaluronic acid methacrylate, oxidized methacrylated alginate, polycaprolactone diacrylate, N,N'-bis(acryloyl)cystamine, N,N'-methylenebis(acrylamide), polyethylene glycol diacrylate, polyethylene glycol dimethacrylate, and combinations thereof.

For example, the coating agent may include one or more selected from the group consisting of polyurethane, nylon, and silicone resin powder.

For example, the viscosity adjuster may include one or more selected from the group consisting of polyvinyl alcohol, polyvinyl acetate, polyvinylpyrrolidone, ammonium acrylate copolymer, polyvinylpyrrolidone/vinyl acetate copolymer, vinyl acetate/styrene copolymer, polyvinylpyrrolidone/styrene copolymer, acrylic/acrylate copolymer, acrylate copolymer, alkyl acrylate copolymer, vinyl acetate/crotonate copolymer, vinyl acetate/vinylpyrrolidone copolymer, alkyl acrylate/vinyl acetate copolymer, acrylate/VA copolymer, guar gum, xanthan gum, cellulose derivatives, polyquaternium, laponite, bentonite, and polyacrylamide polymer.

For example, the skin adhesion sheet may include a dry adhesive layer including a hydrophilic polymer, a coating agent, and a viscosity adjuster without a support layer.

In one embodiment, a content of the coating agent may be less than 50 wt% based on the total weight of the dry adhesive layer. For example, the content of the coating agent may be less than 50 wt%, less than 40 wt%, less than 30 wt%, less than 20 wt%, or less than 10 wt% based on the total weight of the dry adhesive layer, and may be more than 1 wt%, more than 10 wt%, more than 20 wt%, more than 30 wt%, or more than 40 wt%.

In one embodiment, a weight ratio of the hydrophilic polymer and the coating agent may be 1 to 3:1. For example, the weight ratio may be 1:1 or more, 1.5:1 or more, 2:1 or more, or 2.5:1 or more, and may be 3:1 or less, 2.5:1 or less, 2:1 or less, or 1.5:1 or less.

In one embodiment, the dry adhesive layer may further include water and ethanol.

In one embodiment, the skin adhesion sheet may further include a support layer provided on one surface of the dry adhesive layer.

In one embodiment, the support layer may be a polymer resin, and for example, may be made of one or more selected from the group consisting of polyurethane, silicone rubber, styrene-butadiene-styrene copolymer, butyl rubber, latex rubber, spandex, PVC, nylon, and/or hydrogel.

For example, the support layer may be hydrophilic polyurethane.

In one embodiment, the skin adhesion sheet may include a dry adhesive layer including an interpenetrating network of poly(acrylic acid) (PAA) grafted with N-hydroxysuccinimide (NHS) ester and chitosan, and one or more support layers provided on one or both surfaces of the dry adhesive layer.

In one embodiment, the adhesive layer may have a hydrated rubber-state Young's modulus that is lower by two orders of magnitude than the Young's modulus of the dry-state dry adhesive layer.

In one embodiment, the skin adhesion sheet may be a sheet, tape, patch, or film.

In one embodiment, the skin adhesion sheet may be biodegradable.

In one embodiment, the skin adhesion sheet may further include a removable support layer on the other surface of the adhesive layer.

In one embodiment, the skin adhesion sheet may have a moisture content of 1 wt% or less based on the total weight of the skin adhesion sheet.

In one embodiment, the skin adhesion sheet may be a shape-memory sheet.

In one embodiment, the skin adhesion sheet may be a shape-memory sheet that contracts at a memorized ratio after being adhered to skin and after a lapse of time.

In one embodiment, the memorized ratio may be such that, for 150-200% of the surface area at the time of skin adhesion, the surface area after a lapse of time becomes 100%.

In one embodiment, the skin adhesion sheet may be for improving skin wrinkles.

In one embodiment, the skin adhesion sheet may include polyvinyl alcohol (PVA).

In other exemplary embodiments of the present disclosure, a kit for a skin adhesion sheet is provided, which includes the composition described above and a skin adhesion sheet, and is for use by applying the composition onto skin and then attaching the skin adhesion sheet onto the composition.

In one embodiment, the skin adhesion sheet may include a dry adhesive layer.

In one embodiment, the dry adhesive layer may include one or more selected from the group consisting of one or more hydrophilic polymers, one or more amine coupling groups, one or more crosslinkers, a coating agent, and a viscosity adjuster.

For example, the skin adhesion sheet may include a dry adhesive layer including a hydrophilic polymer, a coating agent, and a viscosity adjuster without a support layer.

In one embodiment, the skin adhesion sheet may further include a support layer provided on one surface of the dry adhesive layer.

In one embodiment, the support layer may include a polymer resin.

In one embodiment, the kit for a skin adhesion sheet may be for improving skin wrinkles.

### Specific Examples

Specific Example 1: As a composition for adjusting a skin adhesion force,
the composition is for adjusting an adhesive force and/or a detachment force of a skin adhesion sheet attached to skin, and
the composition includes, based on the total weight of the composition, 5 to 30 wt% of polyol and 60 wt% or less of water.

Specific Example 2: The composition according to Specific Example 1, wherein a weight ratio of the polyol and the water is 3:1 to 1:9.

Specific Example 3: The composition according to Specific Example 1 or 2, wherein the polyol is one or more selected from the group consisting of glycerin, butylene glycol, propylene glycol, and propanediol.

Specific Example 4: The composition is for adjusting an adhesive force of the skin adhesion sheet, and
when the skin adhesion sheet is attached after applying the composition, the adhesive force of the skin adhesion sheet is 100 N/m or more.

Specific Example 5: The composition according to any one of Specific Examples 1 to 4, wherein the composition is for maintaining an adhesion maintenance time of the skin adhesion sheet for 1 hour or longer.

Specific Example 6: The composition according to any one of Specific Examples 1 to 5, wherein the composition is for suppressing skin irritation upon detachment of the skin adhesion sheet, and
the skin irritation upon detachment of the skin adhesion sheet is 1 or less.

Specific Example 7: The composition according to any one of Specific Examples 1 to 6, wherein the composition for adjusting a skin adhesion force is used in an amount of 0.05 to 2 ml per 1 cm³ of the skin adhesion sheet.

Specific Example 8: The composition according to any one of Specific Examples 1 to 7, wherein the composition is applied to the skin before attaching the skin adhesion sheet to the skin.

Specific Example 9: The composition according to any one of Specific Examples 1 to 8, wherein the composition is applied to the skin within 5 seconds before attaching the skin adhesion sheet to the skin.

Specific Example 10: The composition according to any one of Specific Examples 1 to 9, wherein the skin adhesion sheet includes a dry adhesive layer.

Specific Example 11: The composition according to any one of Specific Examples 1 to 10, wherein the dry adhesive layer includes one or more selected from the group consisting of one or more hydrophilic polymers, one or more amine coupling groups, one or more crosslinkers, a coating agent, and a viscosity adjuster.

Specific Example 12: The composition according to any one of Specific Examples 1 to 11, wherein the skin adhesion sheet further includes a support layer provided on one surface of the dry adhesive layer.

Specific Example 13: The composition according to any one of Specific Examples 1 to 12, wherein the support layer includes a polymer resin.

Specific Example 14: The composition according to any one of Specific Examples 1 to 13, wherein the skin adhesion sheet has a moisture content of 1 wt% or less based on the total weight of the skin adhesion sheet.

Specific Example 15: The composition according to any one of Specific Examples 1 to 14, wherein the skin adhesion sheet is a shape-memory sheet.

Specific Example 16: The composition according to any one of Specific Examples 1 to 15, wherein the skin adhesion sheet is for improving skin wrinkles.

Specific Example 17: The composition according to any one of Specific Examples 1 to 16, wherein the skin adhesion sheet includes polyvinyl alcohol (PVA).

Specific Example 18: A kit for a skin adhesion sheet including the composition according to any one of Specific Examples 1 to 17 and
a skin adhesion sheet,
wherein the kit is for use by applying the composition onto skin and then attaching the skin adhesion sheet onto the composition.

Specific Example 19: The kit for a skin adhesion sheet according to Specific Example 18, wherein the skin adhesion sheet includes a dry adhesive layer.

Specific Example 20: The kit for a skin adhesion sheet according to Specific Example 18 or 19, wherein the dry adhesive layer includes one or more selected from the group consisting of one or more hydrophilic polymers, one or more amine coupling groups, one or more crosslinkers, a coating agent, and a viscosity adjuster.

Specific Example 21: The kit for a skin adhesion sheet according to any one of Specific Examples 18 to 20, wherein the skin adhesion sheet further includes a support layer provided on one surface of the dry adhesive layer.

Specific Example 22: The kit for a skin adhesion sheet according to any one of Specific Examples 18 to 21, wherein the support layer includes a polymer resin.

Specific Example 23: The kit for a skin adhesion sheet according to any one of Specific Examples 18 to 22, wherein the kit for a skin adhesion sheet is for improving skin wrinkles.

### [Mode for carrying out invention]

Hereinafter, the present disclosure will be described in more detail with reference to the following embodiments. However, the following embodiments are provided for illustrative purposes only to assist in understanding the present disclosure and are not intended to limit the scope and range of the present disclosure.

### Example

### Example: Preparation of the composition for adjusting a skin adhesion force

According to Tables 1 and 2 below, a composition for adjusting a skin adhesion force was prepared with various contents.

**[Table 1]**

| Unit: wt% | #1 | #2 | #3 | #4 | #5 | #6 | #7 | #8 | #9 | #10 (oil-in-water ) |
|---|---|---|---|---|---|---|---|---|---|---|
| Glycerin | 35 | 15 | 30 | 30 | 25 | 20 | 15 | 15 | 4 | 30 |
| Butylene glycol | 0 | 15 | 0 | 0 | 0 | 0 | 0 | 0 | 0 | 0 |
| Water (distilled water) | 10 | 10 | 10 | 20 | 30 | 40 | 50 | 60 | 65 | 10 |
| EDTA-2Na | 0.05 | 0.05 | 0.05 | 0.05 | 0.05 | 0.05 | 0.05 | 0.05 | 0.05 | 0.05 |
| Betaine | 1.0 | 1.0 | 1.0 | 1.0 | 1.0 | 1.0 | 1.0 | 1.0 | 1.0 | 1.0 |
| Carbomer | 0.1 | 0.1 | 0.1 | 0.1 | 0.1 | 0.1 | 0.1 | 0.1 | 0.1 | 0.1 |
| 1,2-hexanediol | 1.5 | 1.5 | 1.5 | 1.5 | 1.5 | 1.5 | 1.5 | 1.5 | 1.5 | 1.5 |
| Ethylhexylglycerinn | 0.05 | 0.05 | 0.05 | 0.05 | 0.05 | 0.05 | 0.05 | 0.05 | 0.05 | 0.05 |
| Tromethmine | 0.07 | 0.07 | 0.07 | 0.07 | 0.07 | 0.07 | 0.07 | 0.07 | 0.07 | 0.07 |
| Beta glucan | 17.13 | 12.13 | 12.13 | 12.13 | 12.13 | 12.13 | 7.13 | 7.13 | 8.13 | 10 |
| Trehalose | 5 | 5 | 5 | 5 | 5 | 5 | 5 | 5 | 5 | 5 |
| Green tea extract | 15 | 20 | 20 | 15 | 15 | 10 | 10 | 5 | 10 | 10 |
| Sodium hyaluronate | 15 | 20 | 20 | 15 | 10 | 10 | 10 | 5 | 5 | 10 |
| Shea butter | 0 | 0 | 0 | 0 | 0 | 0 | 0 | 0 | 0 | 2 |
| Cetyl alcohol | 0 | 0 | 0 | 0 | 0 | 0 | 0 | 0 | 0 | 1.5 |
| Xanthan gum | 0.1 | 0.1 | 0.1 | 0.1 | 0.1 | 0.1 | 0.1 | 0.1 | 0.1 | 0.1 |
| Glyceryl stearate citrate | 0 | 0 | 0 | 0 | 0 | 0 | 0 | 0 | 0 | 2 |
| Squalane | 0 | 0 | 0 | 0 | 0 | 0 | 0 | 0 | 0 | 10 |
| Total Sum | 100 | 100 | 100 | 100 | 100 | 100 | 100 | 100 | 100 | 100 |

### Example 2: Preparation of the skin adhesion sheet

To prepare the band, chitosan (HMC+ Chitoscience Chitosan 95/500, 95% deacetylation, 2 w/w%), acrylic acid (AAc; 30 w/w%), α-ketoglutaric acid (0.2 w/w%), and poly(ethylene glycol methacrylate) (PEGDMA; Mn = 550, 0.03 w/w%) were dissolved in deionized water. Then, 5 mg of acrylic acid N-hydroxysuccinimide ester (AAc-NHS) was added to 1 mL of the stock solution. Immediately after the addition of AAc-NHS, the mixture was cured in a UV chamber (284 nm, 10 W power) for 30 minutes.

As a non-adhesive polymer support resin, 10 w/w% hydrophilic polyurethane (HydroMed^{™} D3, Advansource Biomaterials) dissolved in an ethanol/water mixture (95:5 v/v) was spin-coated onto the prepared bioadhesive at 400 rpm for 30 seconds and then completely dried. In particular, the thickness of the non-adhesive polymer support could be controlled by adopting different spin-coating conditions. A final band was manufactured by introducing the non-adhesive polymer support resin. The prepared band was sealed in a plastic bag with a desiccant (silica gel packet) and stored at -20 °C before use.

### Example 3: Preparation of the skin adhesion sheet (including PVA)

To prepare the band, polyvinyl alcohol (PVA, 8.5 w/w%) was dissolved together with acrylic acid (AAc; 30 w/w%), α-ketoglutaric acid (0.2 w/w%), and poly(ethylene glycol methacrylate) (PEGDMA; Mn = 550, 0.03 w/w%) in deionized water. Then, 5 mg of acrylic acid N-hydroxysuccinimide ester (AAc-NHS) was added to 1 mL of the stock solution. Immediately after the addition of AAc-NHS, the mixture was cured in a UV chamber (284 nm, 10 W power) for 30 minutes.

As a non-adhesive polymer support resin, 10 w/w% hydrophilic polyurethane (HydroMed^{™} D3, Advansource Biomaterials) dissolved in an ethanol/water mixture (95:5 v/v) was spin-coated onto the prepared bioadhesive at 400 rpm for 30 seconds and then completely dried. In particular, the thickness of the non-adhesive polymer support could be controlled by adopting different spin-coating conditions. A final band was manufactured by introducing the non-adhesive polymer support resin. The prepared band was sealed in a plastic bag with a desiccant (silica gel packet) and stored at -20 °C before use.

### Experimental Example

Subsequent experiments on human subjects were conducted according to the following procedure.
1. Selection criteria of subjects: The experiment was conducted on healthy adults. However, individuals who had psoriasis, acne, eczema, other skin diseases, etc., as well as pregnant or lactating women, or those taking contraceptives, antihistamines, etc., were excluded before the test.
2. Application method: Closed patch test (on the back area).
3. Application amount: 20 µL per chamber (IQ chamber).
4. Application frequency and period: A 48-hour patch test was performed on the back, and evaluations were conducted 30 minutes and 24 hours after patch removal.
5. Reaction evaluation: The evaluation was performed according to the terminology presented in Table 2 below, which represents the present test criteria modified from the CTFA guideline (1981) and Frosch & Kligman (1979). In Table 2 below, 0 indicates no reaction, 1 indicates slight erythema or diffuse, 2 indicates moderate uniform erythema, 3 indicates intense erythema with edema, and 4 indicates intense erythema with edema and vesicle.

**[Table 2]**

| Score | Characteristics |
|---|---|
| 0 | No reaction |
| 1 | Slight erythema, spotty or diffuse |
| 2 | Moderate uniform erythema |
| 3 | Intense erythema with edema |
| 4 | Intense erythema with edema and vesicle |

| | |
|---|---|
| † Grade I (non-irritation range): less than 1 Grade II (mild irritation range): less than 3 Grade III (moderate irritation range): less than 5 Grade IV (strong irritation range): 5 or more | |

The reference photographs for irritation degrees according to the experiment are shown in FIG. 1.

### Experimental Example 1: Measurement of adhesive force according to content

In Table 1, it was confirmed that composition #1 exhibited a decrease in adhesive force, while composition #8 showed an increase in skin adhesion irritation. In addition, the oil-in-water composition #10 did not adhere, and thus the irritation test could not be performed. Specifically, it was confirmed that compositions #1 to #7 corresponded to Grade 1, and composition #8 corresponded to Grade 3.

### Experimental Example 2: Measurement of skin adhesion maintenance time of skin adhesion sheet

It was confirmed that the skin adhesion sheet remained attached to the skin for about 6 to 8 hours in the absence of physical external force or excessive moisture supply. However, in the case of oily skin, the adhesion maintenance time decreased to about 50% due to skin sebum.

### Experimental Example 3: Moisture content data of skin adhesion sheet

As measured using a Thermogravimetric Analyzers (TGA), the moisture content contained in one skin adhesion sheet was confirmed to be less than an average of 0.1 wt%.

### Experimental Example 4: Comparison of use amount between skin adhesion sheet and composition for adjusting skin adhesion force

When 0.01 ml of the composition for adjusting a skin adhesion force was used per 1 cm³ of the skin adhesion sheet, it was confirmed that adhesion was insufficient.

## Claims

1. A composition for adjusting a skin adhesion force, wherein the composition is for adjusting an adhesive force and/or a detachment force of a skin adhesion sheet attached to skin, and the composition comprises, based on the total weight of the composition, 5 to 30 wt% of polyol and 60 wt% or less of water.

2. The composition of claim 1, wherein a weight ratio of the polyol and the water is 3:1 to 1:9.

3. The composition of claim 1, wherein the polyol is one or more selected from the group consisting of glycerin, butylene glycol, propylene glycol, and propanediol.

4. The composition of claim 1, wherein the composition is for adjusting an adhesive force of the skin adhesion sheet, and when the skin adhesion sheet is attached after applying the composition, the adhesive force of the skin adhesion sheet is 100 N/m or more.

5. The composition of claim 1, wherein the composition is for maintaining an adhesion maintenance time of the skin adhesion sheet for 1 hour or longer.

6. The composition of claim 1, wherein the composition is for suppressing skin irritation upon detachment of the skin adhesion sheet, and the skin irritation upon detachment of the skin adhesion sheet is 1 or less.

7. The composition of claim 1, wherein the composition for adjusting a skin adhesion force is used in an amount of 0.05 to 2 ml per 1 cm³ of the skin adhesion sheet.

8. The composition of claim 1, wherein the composition is applied to skin before attaching the skin adhesion sheet to the skin.

9. The composition of claim 1, wherein the composition is applied to skin within 5 seconds before attaching the skin adhesion sheet to the skin.

10. The composition of claim 1, wherein the skin adhesion sheet comprises a dry adhesive layer.

11. The composition of claim 10, wherein the dry adhesive layer comprises one or more selected from the group consisting of one or more hydrophilic polymers, one or more amine coupling groups, one or more crosslinkers, a coating agent, and a viscosity adjuster.

12. The composition of claim 10, wherein the skin adhesion sheet further comprises a support layer provided on one surface of the dry adhesive layer.

13. The composition of claim 12, wherein the support layer comprises a polymer resin.

14. The composition of claim 1, wherein the skin adhesion sheet has a moisture content of 1 wt% or less based on the total weight of the skin adhesion sheet.

15. The composition of claim 1, wherein the skin adhesion sheet is a shape-memory sheet.

16. The composition of claim 1, wherein the skin adhesion sheet is for improving skin wrinkles.

17. The composition of claim 1, wherein the skin adhesion sheet comprises polyvinyl alcohol (PVA).

18. A kit for a skin adhesion sheet, comprising:
the composition according to any one of claims 1 to 17; and
a skin adhesion sheet,
wherein the kit is for use by applying the composition onto skin and then attaching the skin adhesion sheet onto the composition.

19. The kit for a skin adhesion sheet of claim 18, wherein the skin adhesion sheet comprises a dry adhesive layer.

20. The kit for a skin adhesion sheet of claim 18, wherein the dry adhesive layer comprises one or more selected from the group consisting of one or more hydrophilic polymers, one or more amine coupling groups, one or more crosslinkers, a coating agent, and a viscosity adjuster.

21. The kit for a skin adhesion sheet of claim 19, wherein the skin adhesion sheet further comprises a support layer provided on one surface of the dry adhesive layer.

22. The kit for a skin adhesion sheet of claim 21, wherein the support layer comprises a polymer resin.

23. The kit for a skin adhesion sheet of claim 18, wherein the kit for a skin adhesion sheet is for improving skin wrinkles.
